(19) Europäisches Patentamt
European Patent Office
Office européen des brevets

(11) **EP 1 746 931 B1**

(12) **EUROPÄISCHE PATENTSCHRIFT**

(45) Veröffentlichungstag und Bekanntmachung des Hinweises auf die Patenterteilung:
**13.08.2014 Patentblatt 2014/33**

(51) Int Cl.:
***A61B 5/0215*** *(2006.01)*

(21) Anmeldenummer: 05749747.1

(22) Anmeldetag: **17.05.2005**

(86) Internationale Anmeldenummer:
**PCT/EP2005/052258**

(87) Internationale Veröffentlichungsnummer:
**WO 2005/112746 (01.12.2005 Gazette 2005/48)**

(54) **VORRICHTUNG ZUR BESTIMMUNG DES ÜBERGANGS ZWISCHEN SYSTOLE UND DIASTOLE**

DEVICE FOR DETERMINING THE TRANSITION BETWEEN SYSTOLE AND DIASTOLE

DISPOSITIF POUR LA DETERMINATION DE LA TRANSITION ENTRE SYSTOLE ET DIASTOLE

(84) Benannte Vertragsstaaten:
**DE ES FR GB IT**

(30) Priorität: **17.05.2004 DE 102004024335**

(43) Veröffentlichungstag der Anmeldung:
**31.01.2007 Patentblatt 2007/05**

(73) Patentinhaber: **Pulsion Medical Systems AG**
**81829 München (DE)**

(72) Erfinder:
• **PFEIFFER, Ulrich, J.**
**81667 München (DE)**

• **KNOLL, Reinhold**
**81543 München (DE)**
• **REGH, Stephan**
**81673 München (DE)**

(74) Vertreter: **Ascherl, Andreas et al**
**KEHL, ASCHERL, LIEBHOFF & ETTMAYR**
**Patentanwälte - Partnerschaft**
**Emil-Riedel-Strasse 18**
**80538 München (DE)**

(56) Entgegenhaltungen:
**US-A- 5 265 011     US-A1- 2002 022 785**
**US-B1- 6 394 958**

## Beschreibung

Die vorliegende Erfindung betrifft eine Vorrichtung zur Bestimmung des Übergangs zwischen Systole und Diastole innerhalb eines Pulszyklus.

Die Bestimmung des Übergangs zwischen Systole und Diastole (d.h. des Endes der Systole bzw. des Beginns der Diastole) ist insbesondere von Bedeutung bei der Pulskonturanalyse, einer Methode zur Bestimmung hämodynamischer Parameter, vor allem des Herzzeitvolumens (Cardiac Output, CO) und der Schlagvolumenvariation (Stroke Volume Variaten, SVV), aus einem zeitabhängigen Drucksignal, welches dem Aortendruck zumindest näherungsweise entspricht. Die Bestimmung hämodynamischer Parameter mittels Pulskonturanalyse auf Grundlage eines nichtlinearen Windkesselmodells ist in DE 198 14 371 A1 sowie darin aufgeführter weiterführender Literatur ausführlich beschrieben. Ein Pulskonturanalyse-System der Pulsion Medical Systems AG ist unter der Bezeichnung PiCCO kommerziell erhältlich.

Wie in DE 198 14 371 A1 beschrieben, werden für die Berechnung der sogenannten Compliance, welche eine wichtige Größe für die Bestimmung des Herzzeitvolumens darstellt, vorzugsweise nur Druckwerte aus dem Bereich der Diastole verwendet, um das Berechnungsverfahren zu beschleunigen. Dies setzt jedoch voraus, daß der Beginn der Diastole möglichst genau bestimmbar ist.

Im Verlauf der Funktion P(t), d.h. dem zeitlichen Verlauf des der Pulskonturanalyse zugrunde liegenden, dem Aortendruck näherungsweise entsprechenden Drucks, zeigt sich der Übergang zwischen Systole und Diastole als lokales Minimum, welches auch als Dicrotic Notch bezeichnet wird. Der kurze Druckabfall rührt von der Zeit her, welche die Aortenklappe benötigt, um bei beginnender Kontraktion des Herzens zu schließen.

Dieses lokale Ausschwingen der Druckkurve nach unten ist sehr kurz und in den tatsächlich gemessenen Kurven aufgrund von meßtechnisch bedingten Ungenauigkeiten oft wenig ausgeprägt. Je nachdem in welchem Umfang die zugrundeliegenden Druckmessungen verrauscht sind, kann es vorkommen, daß die Lage des Übergangs zwischen Systole und Diastole in der Druckkurve nicht mehr als lokales Minimum auflösbar ist. Dies bedeutet, daß durch Meßrauschen verursachte Ausschläge des Drucksignals in derselben Größenordnung liegen, wie der kurzzeitige Druckabfall am Übergang zwischen Systole und Diastole. In diesem Fall behilft man sich in der Praxis oft damit, den Übergang zwischen Systole und Diastole näherungsweise als Wendepunkt der Funktion P(t) zu bestimmen.

Um von dieser Näherung nur dann Gebrauch machen zu müssen, wenn die direkte Bestimmung des Dicrotic Notch als lokales Minimum nicht mit hinreichender Sicherheit möglich ist, besteht die Möglichkeit, beide Bestimmungsmethoden in einem System zu implementieren. Sobald das System erkennt, daß ein vorgegebener Mindestdruckabfall nicht auflösbar ist, wird auf die näherungsweise Bestimmung des Dicrotic Notch als Wendepunkt der Funktion P(t) zurückgegriffen. D.h. es wird eine Fallunterscheidung vorgenommen, ob ein lokales Minimum klar erkennbar ist oder nicht.

Das Wechseln zwischen zwei Algorithmen kann jedoch in der Praxis zur Folge haben, daß minimale Änderungen der Meßbedingungen deutliche Änderungen der berechneten und angezeigten hämodynamischen Parameter nach sich ziehen: Durch das Wechseln von einer Bestimmungsmethode auf die andere entsteht eine scheinbare Verschiebung des Übergangs zwischen Systole und Diastole, wodurch sich die Datenbasis für alle Parameter ändert, welche ausschließlich aus dem systolischen oder ausschließlich aus dem diastolischen Ast der Funktion P(t) berchnet werden. Durch das "Springen" der Algorithmen wird so womöglich eine physiologische Veränderung vorgetäuscht, welche tatsächlich überhaupt nicht oder nicht im angezeigten Maß vorliegt.

US 2002/0022785 A1 offenbart eine Vorrichtung zur Bestimmung das Übergangs zwischen Systole und Diastole Innerhalb eines Pulsyklus, aufweisend einen Eingangskanal zum Einlesen eines dem Aortendruck eines Patienten zumindest näherungsweise entsprechenden, zeitlich veränderlichen Drucksignals als Funktion der Zelt P(t) und eine Berechnungseinheit welche Differenziermittel zum Bilden der zweiten Ableitung y" aus der Funktion P(t), sowie Auswertemittel zur Ermittlung eines Orts maximaler Krümmung der Funktion P(t) in einem Ermittlungsbereich zwischen dem maximalen und dem minimalen Funktionswert des Pulszyklus als Ort des Übergangs zwischen Systole und Diastole aufweist.

Angesichts der geschilderten Problematik liegt der vorliegenden Erfindung die Aufgabe zugrunde, eine Vorrichtung zu schaffen, welche eine sichere, genaue und gegenüber Störeinflüssen robuste Bestimmung des Obergangs zwischen Systole und Diastole innerhalb eines Pulszyklus ("Notch-Bestimmung") gestattet.

Gemäß einem Aspekt der vorliegenden Erfindung wird diese Aufgabe durch eine Vorrichtung nach Patentanspruch 1 gelößt.

Bevorzugte Ausführungsformen der vorliegenden Erfindung können gemäß einem der Ansprüche 2 bis 11 gestaltet sein.

Nachfolgend wird ein Beispiel einer bevorzugten Ausführungsform der Erfindung anhand der belgefügten, rein schematisch aufzufassenden Zeichnung näher erläutert.

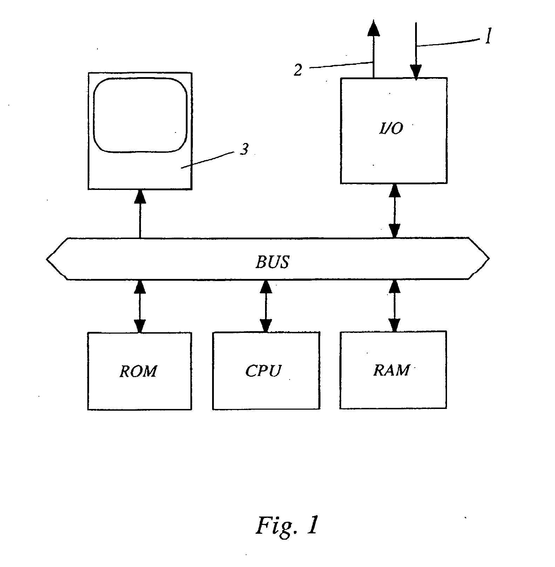

Fig. 1 zeigt dabei ein vereinfachtes Blockschaltbild einer erfindungsgemäßen Vorrichtung.

Die Vorrichtung aus Fig. 1 weist ein Ein-/Ausgabe-Subsystem (I/O) mit mindestens einem Eingangskanal 1 auf, über welchen fortlaufend ein dem Aortendruck eines Patienten zumindest näherungsweise entsprechendes Drucksignal eingelesen wird. Es kann sich dabei um ein analoges Sensor-Signal handeln, welches mittels eines Analog-Digital-Wandlers digitalisiert wird, oder aber es wird bereits ein digitales Signal von einem externen Meßumformer eingelesen.

In der Praxis dient als dem Aortendruck näherungsweise entsprechender Druck ein vortellhafterweise möglichst aortennah über einen arteriellen Katheter gemessener arterieller Druck. Als Meßort kann eine Beinarterie dienen.

Das Ein-/Ausgabe-Subsystem (I/O) kann einen oder mehrere Ausgabe- bzw. Steuerkanäle 2 aufweisen, welche beispielsweise für Kalibrierzwecke verwendbar sind oder aber dem Zusammenwirken mit Peripheriegeräten oder ähnlichem dienen.

Die der Signalverarbeitung dienenden Komponenten der Vorrichtung sind über einen zentralen Bus (BUS) miteinander verbunden.

Das eingelesene Drucksignal wird als Funktion der Zeit P(t) im Arbeitsspeicher (RAM) zwischengespeichert. Die Funktion P (t) wird von der zentralen Recheneinheit (CPU) verarbeitet, um den Übergang zwischen Systole und Diastole sowie ggf. weitere hämodynamische Parameter, wie Herzeitvolumen und Schlagvolumen hieraus zu berechnen. Ein entsprechendes Steuerprogramm, welches die Recheneinheit (CPU) zur Durchführung der entsprechenden Berechnungsschritte veranlaßt, ist in einem Festspeicher (ROM) angelegt.

Die Bestimmung des Übergangs zwischen Systole und Diastole mittels der Recheneinheit (CPU) schließt dabei die folgenden Schritte ein.

Unter Verwendung geeigneter Glättungsalgorithmen werden die erste ($y' = dP/dt$) und die zweite Ableitung ($y'' = d^2P/dt^2$) der Funktion P(t) bestimmt.

Aus diesen wird eine Indikationsfunktion berechnet, welche ein Maß für die lokale Krümmung der Funktion P(t) darstellt. Besonders gut geeignet ist die Krümmungsfunktion

$$K = y''/(1+y'^2)^{3/2}.$$

Diese kann als Kehrwert eines lokalen Krümmungsradius aufgefaßt werden. Verbessert wird die Bestimmung, wenn zuvor eine Achsanpassung vorgesehen ist, welche dem typischen Verlauf einer arteriellen Druckfunktion am Übergang zwischen Systole und Diastole annähernd die Gestalt eines Kreisbogens verleiht. Ein entsprechender Achsanpassungsalgorithmus kann aus empirisch erhobenen Daten gewonnen werden.

Innerhalb des Bereichs der Funktion P(t), in welchem diese Werte von 75 % bis 10 % ihres Maximalwerts innerhalb der aktuellen Pulsperiode annimmt, wird die Lage des Maximums der Krümmungsfunktion K bestimmt. Der entsprechende Zeitpunkt wird gegebenenfalls noch unter Berücksichtigung von Verzögerungsgliedern im Meßaufbau, beispielsweise Filtern, korrigiert.

Liegt das Maximum der Krümmungsfunktion K (ggf. nach dieser Korrektur) innerhalb von 70 % der Dauer der aktuellen Pulsperiode (oder der Dauer einer vorhergehenden Pulsperiode, falls die Berechnung in Echtzeit vor Ende der aktuellen Pulsperiode ausgeführt wird), so wird der Ort des (ggf. korrigierten) Maximums der Krümmungsfunktion K als Zeitpunkt des Übergangs zwischen Systole und Diastole aufgefaßt. Andernfalls wird der Übergang zwischen Systole und Diastole auf 70 % der Dauer der aktuellen Pulsperiode (oder der Dauer einer vorhergehenden Pulsperiode, falls die Berechnung in Echtzeit vor Ende der aktuellen Pulsperiode ausgeführt wird) festgesetzt.

Optional kann noch eine zusätzliche Plausibilitätskontrolle unter Berücksichtigung von Pulsdauer, Ausstoßzeit etc. vorgesehen sein.

Alternativ kann auf die Bestimmung der Krümmungsfunktion verzichtet werden, und anstelle des Maximums der Krümmungsfunktion K das Maximum der zweiten Ableitung y'' der Funktion P(t), ggf. nach entsprechender Korrektur, als Zeitpunkt des Übergangs zwischen Systole und Diastole aufgefaßt werden.

Das Steuerprogramm im Festspeicher (ROM) kann weitere Routinen enthalten, welche die Recheneinheit (CPU) zur Berechnung weiterer hämodynamischer Parameter nach an sich bekannten Algorithmen befähigen.

Über ein Display-Subsystem 3 kann die Funktion P(t) dargestellt und die Lage des Übergangs zwischen Systole und Diastole ausgegeben werden. Zusätzlich oder alternativ hierzu lassen sich weitere hämodynamische Parameter ausgeben.

Selbstredend kann die Vorrichtung mit weiteren dem Fachmann an sich bekannten Komponenten ausgestattet sein, beispielsweise Massenspeichermedien zur Aufzeichnung von Rohdaten und/oder berechneten hämodynamischen Parametern. Die Recheneinheit (CPU) kann mit einem oder mehreren herkömmlichen Mikroprozessoren, ggf. unterstützt durch Coprozessoren zur Beschleunigung von Fließkommaoperationen, aber auch mit sogn. digitalen Signalprozessoren (DSP) ausgestattet sein. Entsprechende Lösungen, wie auch weitere Details der Hardware-Ausführung, können analog üblicher Pulskonturanalyse-Geräte gemäß dem Stand der Technik umgesetzt sein.

**Patentansprüche**

1. Vorrichtung zur Bestimmung des Übergangs zwischen Systole und Diastole innerhalb eines Pulszyklus, aufweisend

   einen Eingangskanal (1) zum Einlesen eines dem Aortendruck eines Patienten zumindest näherungsweise entsprechenden, zeitlich veränderlichen Drucksignals als Funktion der Zeit P(t) und
   eine Berechnungseinheit, welche Differenziermittel zum Bilden der zweiten Ableitung y'' aus der Funktion P(t), sowie Auswertemittel zur Ermittlung eines Orts maximaler Krümmung der Funktion P(t) in einem Ermittlungsbereich zwischen dem maximalen und dem minimalen Funktionswert des Pulszyklus als Ort des Übergangs zwischen Systole und Diastole aufweist,
**dadurch gekennzeichnet, dass** die Vorrichtung ferner Differenziermittel zum Bilden der ersten Ableitung y' aus der Funktion P(t) aufweist,
wobei der Ort maximaler Krümmung für die Auswertemittel als Ort des kleinsten Krümmungsradius

$$(1+y'^2)^{3/2}/y''$$

definiert ist.

2. Vorrichtung gemäß Anspruch 1, welche Speichermittel (RAM) zum Zwischenspeichern des eingelesenen Drucksignals zumindest über den Pulszyklus als Funktion der Zeit P(t) aufweist.

3. Vorrichtung gemäß einem der voranghenden Ansprüche, welche Filtermittel zur Durchführung einer Funktionsglättung aufweist.

4. Vorrichtung gemäß einem der voranghenden Ansprüche, wobei der Ermittlungsbereich sich Innerhalb von 90% bis 10% des maximalen Funktionswerts des Pulszyklus erstreckt.

5. Vorrichtung gemäß Anspruch 4, wobei der Ermittlungsbereich sich innerhalb von 75% bis 10% des maximalen Funktionswerts des Pulszyklus erstreckt.

6. Vorrichtung gemäß einem der vorangehenden Ansprüche, wobei die Auswertemittel ferner Anpassungsmittel zur Achsanpassung für die Funktion P(t) aufweisen, und die Achsanpassung so vorgesehen ist, daß ein aus empirisch erhobenen Daten gewonnener typischer Verlauf einer arteriellen Druckfunktion am Übergang zwischen Systole und Diastole annähernd die Gestalt eines Kreisbogens besitzt.

7. Vorrichtung gemäß einem der vorangehenden Ansprüche, wobei die Auswertemittel ferner Korrekturmittel zur Korrektur der Lage des ermittelten Orts des Übergangs zwischen Systole und Diastole zur Berücksichtigung von Verzögerungsglledern enthalten.

8. Vorrichtung gemäß einem der vorangehenden Ansprüche, wobei die Differenziermittel, Filtermittel und Auswertemittel zumindest teilweise programmtechnisch in Form von Software Implementiert sind.

9. Vorrichtung gemäß einer der vorangehenden Ansprüche, wobei die Berechnungseinhelt Mittel zur Ermittlung mindestens eines weiteren hämodynamischen Parameters aus der Funktion P(t) aufweist.

10. Vorrichtung gemäß Anspruch 9, aufweisend Ausgabemittel (3) zum Ausgeben der hämodynamischen Parameter.

11. Vorrichtung gemäß einem der vorangehenden Ansprüche, ferner aufweisend Anschlußmittel zum Anschluß eines für die Druckmessung geeigneten arteriellen Katheters.

**Claims**

1. Device for determining the transition between systole and diastole within a pulse cycle, comprising an input channel (1) for reading in a pressure signal that can change over time and at least approximately corresponds to the aorta pressure of a patient, as a function of the time P(t),
and a calculation unit that has differentiation means for forming the second derivative y'' from the function P(t), as well as evaluation means for determining a location of maximal curvature of the function P(t) in a determination range between the maximal and the minimal function value of the pulse cycle as the location of the transition between systole and diastole **characterized in that** the device further comprises differentiation means for forming the first derivative y' from the function P(t),
wherein the location of maximal curvature is defined for the evaluation means as the location of the smallest radius of curvature

$$(1+y'^2)^{3/2}/y'' \ .$$

2. Device according to claim 1, which comprises memory means (RAM) for temporary storage of the pressure signal read in, at least over the pulse cycle, as a function of the time P(t).

3. Device according to any of the preceding claims, which comprises filtering means for carrying out function smoothing.

4. Device according to any of the preceding claims, wherein the determination range extends within 90% to 10% of the maximal function value of the pulse cycle.

5. Device according to claim 4, wherein the determination range extends within 75% to 10% of the maximal function value of the pulse cycle.

6. Device according to any of the preceding claims, wherein the evaluation means further comprise adaptation means for axis adaptation of the function P(t), and the axis adaptation is provided in such a manner that a typical progression of an arterial pressure function, obtained from empirically collected data, possesses approximately the shape of a circular arc at the transition between systole and diastole.

**7.** Device according to any of the preceding claims, wherein the evaluation means further comprise correction means for correcting the position of the location determined for the transition between systole and diastole, in order to take delay elements into consideration.

**8.** Device according to any of the preceding claims, wherein the differentiation means, filter means, and evaluation means are implemented, at least in part, in terms of program technology, in the form of software.

**9.** Device according to any of the preceding claims, wherein the calculation unit comprises means for determining at least one additional hemodynamic parameter from the function P(t).

**10.** Device according to claim 9, comprising output means (3) for outputting the hemodynamic parameters.

**11.** Device according to any of the preceding claims, furthermore comprising connection means for connecting an arterial catheter that is suitable for the pressure measurement.

**Revendications**

**1.** Dispositif servant à déterminer la transition entre systole et diastole au sein d'un cycle de pouls, présentant
un canal d'entrée (1) servant à lire un signal de pression correspondant au moins approximativement à la pression de l'aorte d'un patient, variable dans le temps en tant que fonction du temps P(t) et
une unité de calcul, qui présente des moyens de différenciation servant à former la deuxième dérivée y'' à partir de la fonction P(t), ainsi que des moyens d'analyse servant à déterminer un emplacement d'une courbe maximale de la fonction P(t) dans une plage de détermination entre la valeur de fonction maximale et la valeur de fonction minimale du cycle de pouls comme emplacement de la transition entre systole et diastole,
**caractérisé en ce que** le dispositif présente en outre des moyens de différenciation servant à former la première dérivée y' à partir de la fonction P(t), sachant que l'emplacement de la courbe maximale est défini pour les moyens d'analyse en tant qu'emplacement du plus petit rayon de courbe

$$(1 + y'^2)^{3/2}/y''.$$

**2.** Dispositif selon la revendication 1, qui présente des moyens de mémorisation (RAM) servant à mémoriser de manière temporaire le signal de pression lu au moins sur le cycle de pouls comme fonction du temps P(t).

**3.** Dispositif selon l'une quelconque des revendications précédentes, qui présente des moyens de filtre servant à mettre en oeuvre un lissage de fonction.

**4.** Dispositif selon l'une quelconque des revendications précédentes, sachant que la plage de détermination est comprise entre 90 % et 10 % de la valeur de fonction maximale du cycle de pouls.

**5.** Dispositif selon la revendication 4, sachant que la plage de détermination est comprise entre 75 % et 10 % de la valeur de fonction maximale du cycle de pouls.

**6.** Dispositif selon l'une quelconque des revendications précédentes, sachant que les moyens d'analyse présentent en outre des moyens d'adaptation servant à adapter l'axe pour la fonction P(t) et que l'adaptation d'axe est prévue de telle sorte qu'une évolution typique, obtenue à partir des données relevées de manière empirique, d'une fonction de pression artérielle présente, au niveau de la transition entre systole et diastole, approximativement la forme d'un arc de cercle.

**7.** Dispositif selon l'une quelconque des revendications précédentes, sachant que les moyens d'analyse comportent en outre des moyens de correction servant à corriger la position de l'emplacement déterminé de la transition entre systole et diastole afin de prendre en compte des éléments de retardement.

**8.** Dispositif selon l'une quelconque des revendications précédentes, sachant que les moyens de différenciation, les moyens de filtre et les moyens d'analyse sont implémentés au moins en partie, selon une technique de programme, sous la forme de logiciels.

**9.** Dispositif selon l'une quelconque des revendications précédentes, sachant que l'unité de calcul présente des moyens servant à déterminer au moins un autre paramètre hémodynamique à partir de la fonction P(t).

**10.** Dispositif selon la revendication 9, présentant des moyens de sortie (3) servant à délivrer les paramètres hémodynamiques.

**11.** Dispositif selon l'une quelconque des revendications précédentes, présentant en outre des moyens de raccordement servant à raccorder un cathéter artériel approprié pour la mesure de pression.

*Fig. 1*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19814371 A1

- US 20020022785 A1